(19) Europäisches Patentamt
European Patent Office
Office européen des brevets

(11) **EP 4 566 628 A1**

(12) **EUROPEAN PATENT APPLICATION**
published in accordance with Art. 153(4) EPC

(43) Date of publication:
**11.06.2025 Bulletin 2025/24**

(21) Application number: **23850161.3**

(22) Date of filing: **03.08.2023**

(51) International Patent Classification (IPC):
*A61K 45/00* (2006.01)    *A61P 15/00* (2006.01)
*A61P 35/00* (2006.01)    *A61K 31/69* (2006.01)

(52) Cooperative Patent Classification (CPC):
A61K 31/69; A61K 45/00; A61P 15/00; A61P 35/00

(86) International application number:
**PCT/JP2023/028482**

(87) International publication number:
**WO 2024/029608 (08.02.2024 Gazette 2024/06)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **04.08.2022 JP 2022125006**

(71) Applicants:
• **JSR Corporation**
  **Tokyo 105-8640 (JP)**
• **Keio University**
  **Tokyo, 108-8345 (JP)**

(72) Inventors:
• **OOKUBO Aki**
  **Tokyo 105-8640 (JP)**
• **CHIYODA Tatsuyuki**
  **Tokyo 160-8582 (JP)**
• **YOSHIMURA Takuma**
  **Tokyo 160-8582 (JP)**

(74) Representative: **TBK**
**Bavariaring 4-6**
**80336 München (DE)**

(54) **THERAPEUTIC AGENT FOR OVARIAN CLEAR CELL CARCINOMA**

(57)    What is provided is a therapeutic agent that is effective for the treatment of ovarian clear cell carcinoma. A therapeutic agent for ovarian clear cell carcinoma includes, as an active ingredient, a proteasome inhibitor. Furthermore, in the therapeutic agent for ovarian clear cell carcinoma, the proteasome inhibitor is a substance that reversibly or irreversibly binds to a 20s β5 unit of a proteasome and inhibits a chymotrypsin-like activity. Moreover, in the therapeutic agent for ovarian clear cell carcinoma, the proteasome is a 26s proteasome. In addition, in the therapeutic agent for ovarian clear cell carcinoma, a content proportion of the proteasome inhibitor is 80% by mass or more, 90% by mass or more, or 100% by mass.

FIG. 13

EP 4 566 628 A1

**Description**

TECHNICAL FIELD

The present invention relates to a therapeutic agent for ovarian clear cell carcinoma.
Priority is claimed on Japanese Patent Application No. 2022-125006, filed August 4, 2022, the content of which is incorporated herein by reference.

BACKGROUND ART

Since ovarian cancer has few subjective symptoms and the ovary is an intraperitoneal organ, it is extremely difficult to find the ovarian cancer. Thus, in a case where the ovarian cancer is discovered, it is often discovered in an advanced state. For this reason, the 5-year survival rates of patients with ovarian cancer are approximately 40% to 50%, and the survival rates at Stage III or Stage IV have not been improved over the past 40 years.
A majority of ovarian cancers are epithelial ovarian cancers, and several histological types including serous, endometrioid, clear cell, mucinous, transitional, and undifferentiated cancers are known. Among these, ovarian clear cell carcinoma is ovarian cancer with a poor prognosis and is known to have a tendency of being resistant to the treatment due to the unclear etiology.
A main treatment for a patient with ovarian cancer is to firstly remove the cancer by surgical removal, followed by performing standard chemotherapy such as taclipaxel-carboplatin (TC) therapy. However, since ovarian clear cell carcinoma has tumor heterogeneity, it is not possible to completely remove the cancer only by the standard chemotherapy. As a result, there are many cases of recurrence. For this reason, in the treatment of patients with ovarian clear cell carcinoma, it is important to establish second-line or later treatments.
As the second-line treatment, drugs that are effective have been studied by performing genomic analysis on patient-derived ovarian cancer tissues. However, it is known that the probability of discovering a gene mutation through genomic analysis is approximately 50%, and the probability of finding a drug that is expected to be effective therefrom is approximately 10%. It is expected that the effectiveness to the drug is related to not only the gene mutation but also drug metabolism of the cancer tissue and other gene expressions. For this reason, in addition to the genomic analysis, it is considered that the probability of finding a drug with a possibility of effectiveness can be increased by examining the drug sensitivity of the cancer tissue and the like using the ovarian tissue of the patient or a tissue functionally close to the ovarian tissue.
In Patent Document 1 or Non-Patent Document 1, the drug sensitivity is examined using an ES-2 cell line which has sometimes been considered as an ovarian clear cell carcinoma cell line in the related art. However, as shown in Non-Patent Documents 2 and 3, it has been revealed that the ES-2 cell line is not an ovarian clear cell carcinoma tissue but is an origin of other ovarian cancers such as serous ovarian cancer tissues, from immunohistochemical analysis, qPCR analysis, and mutation profile thereof.

Citation List

Patent Document

Patent Document 1: United States Patent Application, Publication No. 2014/0364375

Non-Patent Documents

Non-Patent Document 1: PLoS One. 2020 Jan 15;15(1):e0227727. "Structure-function analyses of candidate small molecule RPN13 inhibitors with antitumor properties"
Non-Patent Document 2: J Clin Pathol. 2014 Oct; 67(10):921-2. "Caution over use of ES2 as a model of ovarian clear cell carcinoma"
Non-Patent Document 3: Nat Commun. 2013; 4:2126. "Evaluating cell lines as tumour models by comparison of genomic profiles"

SUMMARY OF INVENTION

Technical Problem

An object of the present invention is to provide a therapeutic agent that is effective for the treatment of ovarian clear cell carcinoma.

Solution to Problem

The present invention includes the following aspects.

[1] A therapeutic agent for ovarian clear cell carcinoma, including, as an active ingredient: a proteasome inhibitor.
[2] The therapeutic agent for ovarian clear cell carcinoma according to [1], in which the proteasome inhibitor is a substance that reversibly or irreversibly binds to a 20s β5 unit of a proteasome and inhibits a chymotrypsin-like activity.
[3] The therapeutic agent for ovarian clear cell carcinoma according to [1], in which the proteasome inhibitor is a substance that irreversibly binds to a 20s β5 unit of a proteasome and inhibits a chymotrypsin-like activity.
[4] The therapeutic agent for ovarian clear cell carcinoma according to [2] or [3], in which the proteasome is a 26s proteasome.
[5] The therapeutic agent for ovarian clear cell carcinoma according to any one of [1] to [4], in which a content proportion of the proteasome inhibitor included in the active ingredient is 80% by mass or more, 90% by mass or more, or 100% by mass.
[6] The therapeutic agent for ovarian clear cell carcinoma according to any one of [1] to [5], in which the ovarian clear cell carcinoma has cisplatin resistance, carboplatin resistance, or both the cisplatin resistance and the carboplatin resistance.
[7] The therapeutic agent for ovarian clear cell carcinoma according to any one of [1] to [6], in which the ovarian clear cell carcinoma expresses calnexin, ero1La, CHOP, PDI, PERK, BiP, or IRE1α.
[8] The therapeutic agent for ovarian clear cell carcinoma according to [7], in which the ovarian clear cell carcinoma expresses calnexin and PDI.

Advantageous Effects of Invention

According to the present invention, it is possible to provide a pharmaceutical composition that is effective for the treatment of ovarian clear cell carcinoma.

BRIEF DESCRIPTION OF DRAWINGS

[FIG. 1] A photographic view showing a microphotographic view of cell aggregates in the culture of the present Example.
[FIG. 2] A photographic view of formalin-fixed paraffin-embedded (FFPE) sections of a tumor and an organoid of the present Example, which are stained with hematoxylin and eosin (HE), and subjected to p53 immunostaining.
[FIG. 3] (a) is a view showing the results of NGS gene mutation analysis, and (b) is a graphic view showing the results of analyzing the commonality of gene mutations from the results of the NGS gene mutation analysis.
[FIG. 4] A graphic view showing the results of the allele frequency of gene mutations.
[FIG. 5] A graphic view showing the results of the change in number of copies of chromosomes.
[FIG. 6] A view showing the results of exosome sequencing of ovarian clear cell carcinoma organoids in 11 cases.
[FIG. 7] A graphic view showing the results of the cell viability for a sensitivity test of ovarian cancer organoids to olaparib or cisplatin.
[FIG. 8] (a) is a photographic view of a tissue of an immunodeficient mouse to which an ovarian cancer organoid has been transplanted, (b) is a photographic view showing an HE staining view of CCC19-042 of a clear cell carcinoma organoid, (c) is a photographic view showing an HNF1β staining view of CCC19-042 of the clear cell carcinoma organoid, (d) is a photographic view showing an HE staining view of HGSC18-016 of a serous carcinoma organoid, and (e) is a photographic view showing a p53 staining view for HGSC18-016 of the serous carcinoma organoid.
[FIG. 9] (a) is a schematic view of a method for constructing high-throughput drug screening (HTDS), and (b) is a photographic view of seeding of a cell suspension in the construction of HTDS.
[FIG. 10] A graphic view of the results of HTDS.
[FIG. 11] A schematic view of a protocol for screening of HTDS using an ovarian clear cell carcinoma organoid.
[FIG. 12] A view showing a heat map based on the results of screening of HTDS using an ovarian clear cell carcinoma organoid.

[FIG. 13] A graphic view showing the results of measuring the cell viability by measuring a proteasome inhibitor-sensitive ATP activity of an ovarian clear cell carcinoma organoid.

[FIG. 14] A graphic view showing the results of measuring the cell viability for another sample of FIG. 13.

[FIG. 15A] A microscopic observation image of an ovarian clear cell carcinoma organoid CCC19-042.

[FIG. 15B] (i) is a tissue image after the transplantation of the ovarian clear cell carcinoma organoid CCC19-042 into an immunodeficient mouse, (ii) is an HE staining view of the ovarian clear cell carcinoma organoid CCC19-042 transplanted into an immunodeficient mouse, and (iii) is an HNF1β immunostaining view of the ovarian clear cell carcinoma organoid CCC19-042 transplanted into an immunodeficient mouse.

[FIG. 15C] A graphic view showing a change in tumor volume in a case where a vehicle and bortezomib are administered to an immunodeficient mouse transplanted with the ovarian clear cell carcinoma organoid.

[FIG. 16] (a) is a view schematically showing a single-sample gene set enrichment analysis (ssGSEA), and (b) is a graphic view showing the results of the ssGSEA for gene expression in an inositol-requiring enzyme 1α (IRE1α) pathway, a PKR-like endoplasmic reticulum kinase (PERK) pathway, and an activating transcription factor (ATF) pathway.

[FIG. 17] A graphic view showing the results of measuring the cell viability for cyclin-dependent kinase (CDK) 1, 2, 5, and 9 inhibitors.

DESCRIPTION OF EMBODIMENTS

Hereinafter, the therapeutic agent according to the present invention will be described with reference to embodiments. It should be noted that the present invention is not limited to the following embodiments. In the present specification, a notation showing a numerical value range such as "1 to 1,000" has the same meaning as "1 or more and 1,000 or less". In addition, a notation including a plurality of terms "or more" and "or less" such as "1 to 1,000 and preferably 10 to 100" has the same meaning as "1 or more and 1,000 or less, 1 or more and 100 or less, 10 or more and 1,000 or less, or 10 or more and 100 or less".

[Therapeutic Agent for Ovarian Clear Cell Carcinoma]

The therapeutic agent for ovarian clear cell carcinoma of the present embodiment contains, as an active ingredient, a proteasome inhibitor.

Here, the proteasome inhibitor broadly refers to a substance that has an action of inhibiting a proteasome. The proteasome is an enzyme complex involved in protein degradation. As the proteasome, a 26s proteasome in which two 19s (19s particles) are bound to 20s (20s proteasome, core particle) having a protease activity is known. In addition, a form in which 11s (11s particles) are bound to 20s is also known. In the present embodiment, in a case of being simply referred to the proteasome, it is intended to include not only the 26s proteasome but also complexes constituting the 20s, 19s, and 11s, and a part of components thereof. The proteasome inhibitor broadly includes a component that interacts with these proteasomes and inhibits an action thereof. A proteasome inhibitor, for example, bortezomib is generally used for the treatment of multiple myeloma, as will be described later.

Examples of the proteasome inhibitor include bortezomib (CAS RN (registered trademark): 179324-69-7), MG-132 (CAS RN (registered trademark): 133407-82-6), carfilzomib (CAS RN (registered trademark): 868540-17-4), ixazomib citrate (CAS RN (registered trademark): 1239908-20-3), ixazomib (CAS RN (registered trademark): 1072833-77-2), ONX-0914 (CAS RN (registered trademark): 960374-59-8), oprozomib (CAS RN (registered trademark): 935888-69-0), delanzomib (CAS RN (registered trademark): 847499-27-8), celastrol (CAS RN (registered trademark): 34157-83-0), epoxomicin (CAS RN (registered trademark): 134381-21-8), shikonin (CAS RN (registered trademark): 517-89-5), VR23 (CAS RN (registered trademark): 1624602-30-7), isoginkgetin (CAS RN (registered trademark): 548-19-6), salinosporamide A (CAS RN (registered trademark): 437742-34-2), RA-190 (CAS RN (registered trademark): 1617495-03-0), PI-1840 (CAS RN (registered trademark): 1401223-22-0), and acetylcorynoline (CAS RN (registered trademark): 18797-80-3), as well as physiologically acceptable salts thereof.

The proteasome inhibitor of the present embodiment is preferably a substance that reversibly or irreversibly binds to the 20s β5 unit of the proteasome. Examples of the substance that reversibly or irreversibly binds to the 20s β5 unit of the proteasome include, among the above-described proteasome inhibitors, bortezomib, carfilzomib, ixazomib citrate, ixazomib, oprozomib, and salinosporamide A. Among the substances that reversibly or irreversibly bind to the 20s β5 unit of the proteasome, bortezomib, carfilzomib, or ixazomib is preferable, and bortezomib or ixazomib is more preferable.

In addition, the proteasome inhibitor of the present embodiment is more preferably a substance that irreversibly binds to the 20s β5 unit of the proteasome.

Examples of the substance that irreversibly binds to the 20s β5 unit of the proteasome include carfilzomib and oprozomib.

A reason why the substance that reversibly or irreversibly binds to the 20s β5 unit of the proteasome is effective is presumed to be that only the β1, β2, and β5 units have a proteolytic activity, and the β5 unit is related to a rate-limiting step thereof.

The proteasome inhibitor of the present embodiment may be an inhibitor of any one of β5 having a chymotrypsin-like activity or β1 having a caspase-like activity among the 20s subunits. Here, examples of the proteasome inhibitor include a proteasome inhibitor that reversibly or irreversibly binds to and inhibits β5 or β1, but a substance that binds to β5 and inhibits a chymotrypsin-like activity is preferable since it more strongly inhibits the activity.

The proteasome inhibitor of the present embodiment is preferably a substance that inhibits a chymotrypsin-like activity.

In general, it is considered that the chymotrypsin-like activity of the proteasome is carried out by a β1i unit and a β5i unit as specific functions, in addition to the β5 unit of the β type subunit. Therefore, the substance that inhibits a chymotrypsin-like activity is preferably a substance that inhibits the function of the β1i unit and the β5i unit, in addition to the β5 unit, and for example, it is also preferably a substance that reversibly or irreversibly binds to the β1i unit and the β5i unit, in addition to the β5 unit.

Examples of the substance that inhibits a chymotrypsin-like activity include bortezomib, carfilzomib, ixazomib citrate, ixazomib, delanzomib, celastrol, VR23, isoginkgetin, and PI-1840, among the above-described proteasome inhibitors.

A reason why the substance that inhibits a chymotrypsin-like activity is effective is presumed to be that the chymotrypsin-like activity of the proteasome β5 subunit (PSMB5) is related to a rate-limiting step for protein degradation.

It is also preferable that the proteasome is a 26s proteasome in the therapeutic agent for ovarian clear cell carcinoma of the present embodiment.

In the therapeutic agent for ovarian clear cell carcinoma of the present embodiment, the content proportion of the proteasome inhibitor included in the active ingredient may be 70% by mass or more, 75% by mass or more, 80% by mass or more, 85% by mass or more, 90% by mass or more, 95% by mass, or 100% by mass or more. The term "active ingredient" in the present specification refers to a substance that exhibits a physiological activity among the substances included in the therapeutic agent for ovarian clear cell carcinoma.

The therapeutic agent for ovarian clear cell carcinoma of the present embodiment can be used for the treatment of ovarian clear cell carcinoma. The ovarian clear cell carcinoma is generally said to have a low sensitivity to an anticancer agent, making it relatively difficult to progress, but difficult to treat.

In addition, the ovarian clear cell carcinoma is pathologically characterized by clear cells or Hobnail-like cells, and the clear cells have a bright and wide cytoplasm that includes glycogen. Immunohistochemically, the ovarian clear cell carcinoma is positive for a glucose metabolism-related transcription factor (hepatocyte nuclear factor-1β, HNF-1β), or the like.

In the therapeutic agent for ovarian clear cell carcinoma of the present embodiment, the ovarian clear cell carcinoma may have cisplatin resistance, carboplatin resistance, or both the cisplatin resistance and the carboplatin resistance.

Here, cisplatin or carboplatin is a drug that is used for the treatment, prevention, and symptom relief of various types of tumors and cancers and is also used for chemotherapy of ovarian cancer. Cisplatin or carboplatin is also used for the treatment of ovarian clear cell carcinoma.

However, the ovarian clear cell carcinoma cells may have cisplatin resistance or carboplatin resistance by acquiring resistance through a treatment with cisplatin. The therapeutic agent for ovarian clear cell carcinoma of the present embodiment can be used for the treatment even in a case where the ovarian clear cell carcinoma has cisplatin resistance, carboplatin resistance, or both cisplatin resistance and carboplatin resistance, and is thus effective for these uses.

In the therapeutic agent for ovarian clear cell carcinoma of the present embodiment, it is preferable that the ovarian clear cell carcinoma expresses calnexin, an endoplasmic reticulum oxidoreductase 1α (ero1La), a C/EBP homologous protein (CHOP), a protein disulfide isomerase (PDI), a PKR-like endoplasmic reticulum kinase (PERK), a binding immunoglobulin protein (BiP), an inositol-requiring enzyme 1α (IRE1α), or any combination thereof.

Here, the factor expressed by the ovarian clear cell carcinoma may be calnexin (NCBI Reference Sequence (RefSeq): XP_031293834.1 and the like), ero1La (RefSeq: NP_001369396.1 and the like), CHOP (RefSeq: XP_047284402.1 and the like), PDI (RefSeq: XP_009308609.1 and the like), PERK (RefSeq: XP_023807016.1 and the like), BiP, or IRE1α (RefSeq: NP_005071.2 and the like).

In addition, it is particularly preferable that the ovarian clear cell carcinoma expresses calnexin and PDI.

[Pharmaceutical Composition]

The therapeutic agent for ovarian clear cell carcinoma of the present embodiment is suitably used for the treatment of ovarian clear cell carcinoma. That is, the therapeutic agent for ovarian clear cell carcinoma of the present embodiment can also be referred to as a pharmaceutical composition used for the treatment of ovarian clear cell carcinoma.

In addition, the therapeutic agent for ovarian clear cell carcinoma can also be formulated as a pharmaceutical composition including other components. The pharmaceutical composition for the treatment of ovarian clear cell carcinoma may also appropriately include various components contained in other pharmaceutical compositions known in the related art.

A form of the pharmaceutical composition of the present embodiment is not particularly limited, and can be, for example, a dispersion body such as a solution, a sol, and a gel, or a powder. The pharmaceutical composition can be administered orally in the form of, for example, a tablet, a capsule, an elixir, or the like, or non-orally in the form of, for example, an enema.

As the pharmaceutically acceptable carrier, those usually used for the formulation of a pharmaceutical composition can be used without particular limitation. More specific examples thereof include binders such as gelatin, cornstarch, gum tragacanth, and gum arabic; excipients such as starch and crystalline cellulose; swelling agents such as alginic acid; and solvents such as water, ethanol, and glycerin.

The pharmaceutical composition of the present embodiment may include additives. Examples of the additives include lubricants such as calcium stearate and magnesium stearate; sweeteners such as sucrose, lactose, saccharin, and maltitol; flavorants such as a peppermint and a red oil; stabilizers such as benzyl alcohol and phenol; buffers such as phosphate and sodium acetate; dissolution aids such as benzyl benzoate and benzyl alcohol; antioxidants; and preservatives.

The pharmaceutical composition of the present embodiment can be formulated by appropriately combining the above-described proteasome inhibitor, the above-described pharmaceutically acceptable carrier, and the above-described additives and mixing them in a form of a unit dose required for generally accepted pharmaceutical practice.

The dose of the pharmaceutical composition varies depending on the type, method of administration, and dose of the drug, or the symptoms, body weight, age, sex, and the like of the patient, and thus, cannot be determined in a general manner. However, the proteasome inhibitor in the active ingredient included in the pharmaceutical composition is usually 0.3 mg/m$^2$ body surface area to 550 mg/m$^2$ body surface area per day.

With regard to bortezomib, in the case of intravenous administration, it is considered appropriate to administer the active ingredient, for example, at 0.3 mg/m$^2$ body surface area to 2.5 mg/m$^2$ body surface area, preferably, for example, 0.7 mg/m$^2$ body surface area to 2.3 mg/m$^2$ body surface area, and more preferably 1 mg/m$^2$ body surface area to 2 mg/m$^2$ body surface area per day.

With regard to ixazomib, in a case of intravenous administration, it is considered appropriate to administer the active ingredient, for example, at 1 mg/m$^2$ body surface area to 550 mg/m$^2$ body surface area, preferably, for example, 0.5 mg/m$^2$ body surface area to 270 mg/m$^2$ body surface area, and more preferably 1 mg/m$^2$ body surface area to 3 mg/m$^2$ body surface area per day.

With regard to carfilzomib, in a case of intravenous administration, it is considered appropriate to administer the active ingredient, for example, at 10 mg/m$^2$ body surface area to 100 mg/m$^2$ body surface area, preferably 15 mg/m$^2$ body surface area to 80 mg/m$^2$ body surface area, and more preferably 20 mg/m$^2$ body surface area to 56 mg/m$^2$ body surface area per day.

(Effects of Present Embodiment)

According to the therapeutic agent for ovarian clear cell carcinoma of the present embodiment, by containing a proteasome inhibitor as the active ingredient, a therapeutic agent effective for the treatment of ovarian clear cell carcinoma can be obtained.

As shown in Examples, the present inventors established a cell line and a culture method for an ovarian cancer organoid. Furthermore, the present inventors performed drug screening using an ovarian clear cell carcinoma cell line (ovarian clear cell carcinoma organoid), and verified the cell viability of a candidate drug. As a result, it was found that the proteasome inhibitor was effective for the clear cell carcinoma organoid.

In the previous studies, there have been reports that Kuramochi, OVSAHO, and SNU119, which are cell lines considered to be high-grade serous carcinoma, were transplanted into immunodeficient mice, but tumor formation was not observed (Mitra AK et al., Gynecol Oncol. 2015). Therefore, there has been a demand for an ovarian cancer cell line that can also be suitably used for in vivo research on tumors.

As shown in Examples which will be described later, the present inventors established an ovarian clear cell carcinoma organoid, transplanted it into an immunodeficient mouse, and confirmed tumor formation, pathological features, and HNF1-β positivity.

With regard to a fact that the proteasome inhibitor is effective for the treatment of ovarian clear cell carcinoma, the ovarian clear cell carcinoma is a cancer in which multiple mutations of tumor heterogeneity are observed. Here, since the proteasome plays essential functions as a housekeeping molecule for maintaining protein homeostasis in all cells, it is considered that the proteasome inhibitor may have been effective for the treatment of ovarian clear cell carcinoma since it can function as a target even in the presence of multiple mutations such as those in the ovarian clear cell carcinoma.

[Method for Treating Ovarian Clear Cell Carcinoma]

The proteasome inhibitor of the present embodiment can be used in a method for treating ovarian clear cell carcinoma, the method including administering an effective amount of a proteasome inhibitor to a patient.
The method for treating ovarian clear cell carcinoma broadly includes a method for treating a subject suffering from ovarian clear cell carcinoma, as well as a method for preventing ovarian clear cell carcinoma, a treatment method for prognosis of ovarian clear cell carcinoma, and the like. The patient broadly includes, among humans and other animals, a subject who has suffered from the above-described ovarian clear cell carcinoma and a subject who is treated for prevention or prognosis.
The effective amount of the proteasome inhibitor to be administered is the same as that described for the dose of the pharmaceutical composition.

[Other Aspects of Present Embodiment]

Another aspect of the present embodiment is a proteasome inhibitor for use in the treatment of ovarian clear cell carcinoma.
The constitution and the using method of the proteasome inhibitor can be selected from the above-described ones.

Examples

Examples are shown below. In addition, the present invention is not limited to Examples.
All experiments were performed on pathological specimens of patients with ovarian cancer, who had been explained and had agreed, in accordance with an ethical research plan approved by the Ethics Committee of Keio University School of Medicine.

(Establishment of Ovarian Cancer Organoid)

[Experimental Example 1: Preparation of Cell Aggregates]

Establishment of an ovarian clear cell carcinoma organoid (CCCO) from a specimen of ovarian clear cell carcinoma (CCC) was performed.
CA18-015, a lesion tissue name, was collected as a pathological specimen from a patient with ovarian cancer. The histological type of CA18-015 was clear cell carcinoma. Under a sterile operation, the ovarian cancer tissue was placed in a centrifuge tube containing a cell preservation solution (product name "Advanced DMEM/F-12", manufactured by Thermo Fisher Scientific, Inc.), and transported to a laboratory in a cooled state on ice. In a safety cabinet, the cell preservation solution used during transportation was removed, and the ovarian cancer tissue was washed three times by mixing under inversion of 20 ml of an ice-cooled washing solution (product name "HBSS, no calcium, no magnesium", manufactured by Thermo Fisher Scientific, Inc.).
The ovarian cancer tissue was transferred to a 9 cm dish for tissue culture (product name, "Petri Dish 90Φ for Cell Culture", manufactured by Sumitomo Bakelite Co., Ltd.). This culture dish was placed on ice and the necrotic tissue was removed in the dish using an ophthalmic surgical blade. The ovarian cancer tissue piece from which the necrotic tissue had been removed was fragmented to about 0.5 to 1 mm square using ophthalmic surgical blade. The fragmented ovarian cancer tissue piece and 45 mL of the washing solution were transferred to a 50 mL centrifuge tube and centrifuged, the supernatant was discarded, 45 mL of the washing solution was added thereto, and the mixture was mixed under inversion.
10 mL of the cell dispersion liquid was added per mass of 600 mg of the fragmented ovarian cancer tissue piece and mixed. The container containing the fragmented ovarian cancer tissue piece was shaken in a shaking water bath at 37°C and 160 rpm for 30 minutes, and the contents of the container were passed through a cell strainer having a mesh size of 100 μm. Next, 40 mL of the washing solution was added thereto and centrifuged, the supernatant was discarded, then 40 mL of the washing solution was added thereto and centrifuged, and the supernatant was discarded.
As the cell dispersion liquid, a liquid obtained by adding 1.1 mL of collagenase (final concentration: 1 mg/mL), 0.55 mL of Dispase II (final concentration: 3.6 mg/mL), Y-27136 (final concentration: 10 μM), and 100 U of DNase I (Roche, 100 Units/10 mL) to 8.5 mL of the washing solution was used.
A hemolytic agent (product name "ACK Lysing Buffer", manufactured by Thermo Fisher Scientific, Inc.) was mixed with the cell aggregates after the dispersion treatment, and the mixture was allowed to stand for 5 minutes and subjected to a hemolysis treatment. Next, 40 mL of a washing solution was added thereto and centrifuged (centrifugal force RCF: 400 × g, centrifugal time: 5 minutes), the supernatant was discarded, 40 mL of a washing solution was added thereto and centrifuged (centrifugal force RCF: 400 × g, centrifugal time: 5 minutes), the supernatant was discarded, a cell preservation solution was added thereto and centrifuged (centrifugal force RCF: 400 × g, centrifugal time: 5 minutes), and the supernatant was discarded, thereby obtaining cell aggregates having a cell diameter of 11 $\mu$m or more.

From the same operation, cell aggregates were obtained from the following pathological specimens as pathological specimens of ovarian clear cell carcinoma from patients with ovarian cancer: lesion tissue names CA18-148, CA19-001, CA19-010, CA19-042, CA19-044, CA19-055, CA19-076, CA19-078, and CA19-079; from the following pathological specimens as pathological specimens of serous ovarian cancer: lesion tissue names CA18-016, CA18-036, and CA18-064; and from the following pathological specimens as pathological specimens of endometrioid ovarian cancer: CA18-053, CA18-055, and CA18-065.

[Experimental Example 2: Establishment of Ovarian Clear Cell Carcinoma Cell Line (Ovarian Clear Cell Carcinoma Organoid)]

3,000 cell aggregates obtained from CA18-015 were seeded in a 48-well plate together with 25 $\mu$L of Matrigel (registered trademark, manufactured by Corning Inc.) thawed under ice-cooling, and allowed to stand in a $CO_2$ incubator (37°C, 5% by volume $CO_2$) for 10 minutes to allow the Matrigel (manufactured by Thermo Fisher Scientific, Inc.) to be gelled. 200 to 300 $\mu$L of a culture medium containing a B27 serum-free supplement (manufactured by Thermo Fisher Scientific, Inc.), Wnt3A, EGF, R-spondin, and Noggins was added to each well, and the cells were cultured in a $CO_2$ incubator (37°C, 5% by volume of $CO_2$) for 14 days. In the culture, the culture medium was replaced every 2 or 3 days from seeding. The obtained culture product was able to be subcultured and an ovarian clear cell carcinoma organoid was established. This ovarian clear cell carcinoma organoid was designated as CCC18-015.

In addition, in the same operation, as ovarian clear cell carcinoma organoids, CCC18-148, CCC19-001, CCC19-010, CCC19-042, CCC19-044, CCC19-055, CCC19-076, CCC19-078, and CCC19-079 were each obtained from cell aggregates obtained from CA18-148, CA19-001, CA19-010, CA19-042, CA19-044, CA19-055, CA19-076, CA19-078, and CA19-079; as serous ovarian cancer organoids, HGSC18-016, HGSC18-036, and HGSC18-064 were each obtained from cell aggregates obtained from CA18-016, CA18-036, and CA18-064; and as endometrioid ovarian cancer organoids, EM18-053, EM18-055, and EM18-065 were each obtained from cell aggregates obtained from CA18-053, CA18-055, and CA18-065.

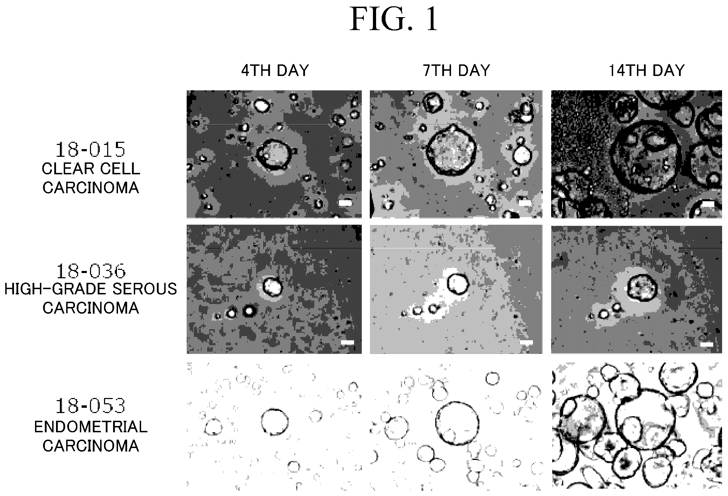

FIG. 1 shows a microphotographic view of cell aggregates in culture. Photographic views of EM18-053, EM18-055, and EM18-065 on each of the 4th, 7th, and 14th days are shown.

The scale in the drawing is 100 $\mu$m, and the observation was performed using an all-in-one fluorescence microscope BZ-X700 (Keyence Corporation).

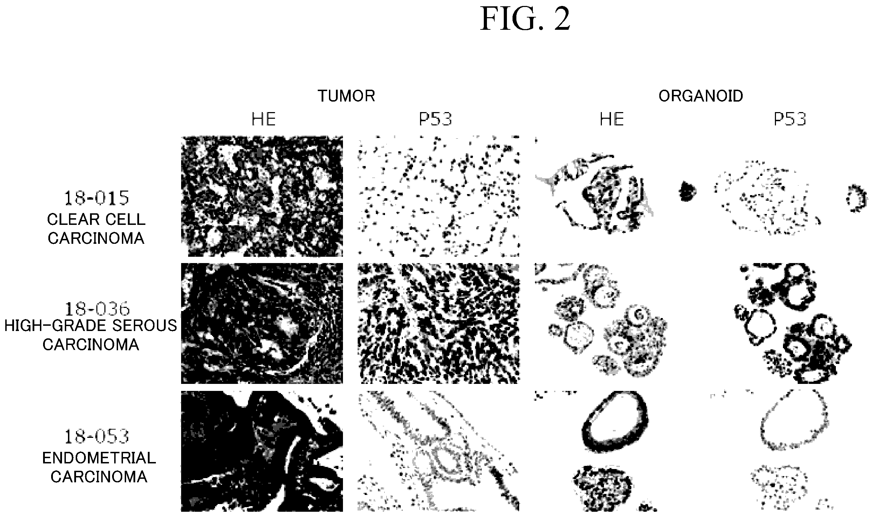

FIG. 2 shows images of thinly sliced slides that were created from FFPE sections of tumors and organoids and subjected to HE staining and p53 immunostaining, and shows each of a tumor and an organoid (CCC18-015) of 18-015, a tumor and an organoid (HGS18-036) of 18-036, and a tumor and an organoid (END18-053) of 18-053. Among these, 18-015 is clear cell carcinoma and an organoid thereof (CCCO).

The scale in the drawing is 100 $\mu$m, and the observation was performed using an all-in-one fluorescence microscope BZ-X700 (Keyence Corporation).

[Experimental Example 3: Evaluation of Ovarian Clear Cell Carcinoma Organoid]

DNA was extracted from the pathological specimen and the ovarian cancer organoid obtained therefrom, the exon sequence was enriched using an exome capture kit, a library (1053 gene panel) was prepared, and gene mutation analysis was performed using NGS (Illumina, Inc.).

For each of HGSC18-016, HGSC18-064, and HGSC18-036 of high-grade serous carcinoma, CCC18-015 of clear cell carcinoma, and EM18-053, EM18-055, and EM18-065 of endometrial carcinoma, analysis of the tumor (T) and the organoid (O) was performed.

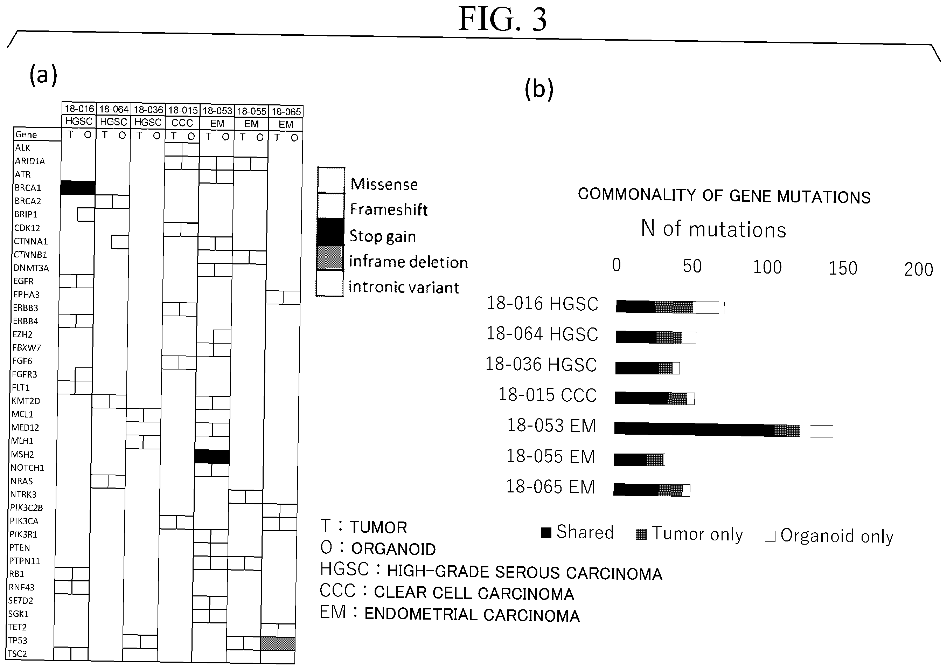

FIG. 3 shows the results of the gene mutation analysis. FIG. 3(a) is the result of the gene mutation analysis of NGS, and FIG. 3(b) shows the commonality of the gene mutation between T and O for each cell line. In any of the cell lines, the number (N) of mutations of approximately 20 to 30 was common in T and O. In particular, in EM18-053, the number (N) of mutations of 100 or more was common.

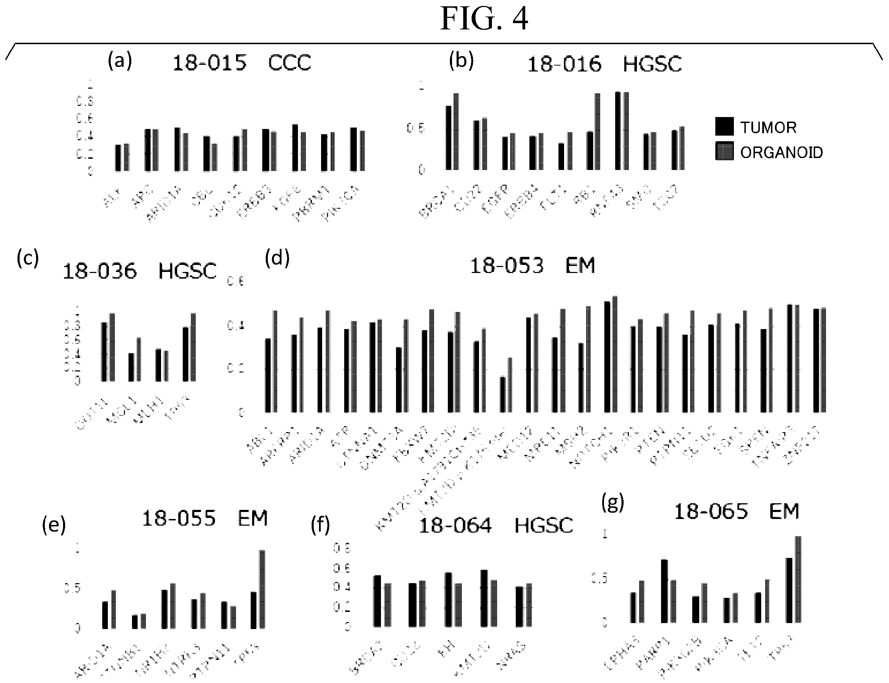

FIG. 4 is a graphic view showing the results of the allele frequency of the gene mutation. The results of CCC18-015 in FIG. 4(a), HGSC18-016 in FIG. 4(b), HGSC18-036 in FIG. 4(c), EM18-053 in FIG. 4(d), EM18-055 in FIG. 4(e), HGSC18-064 in FIG. 4(f), and EM18-065 in FIG. 4(g) are shown.

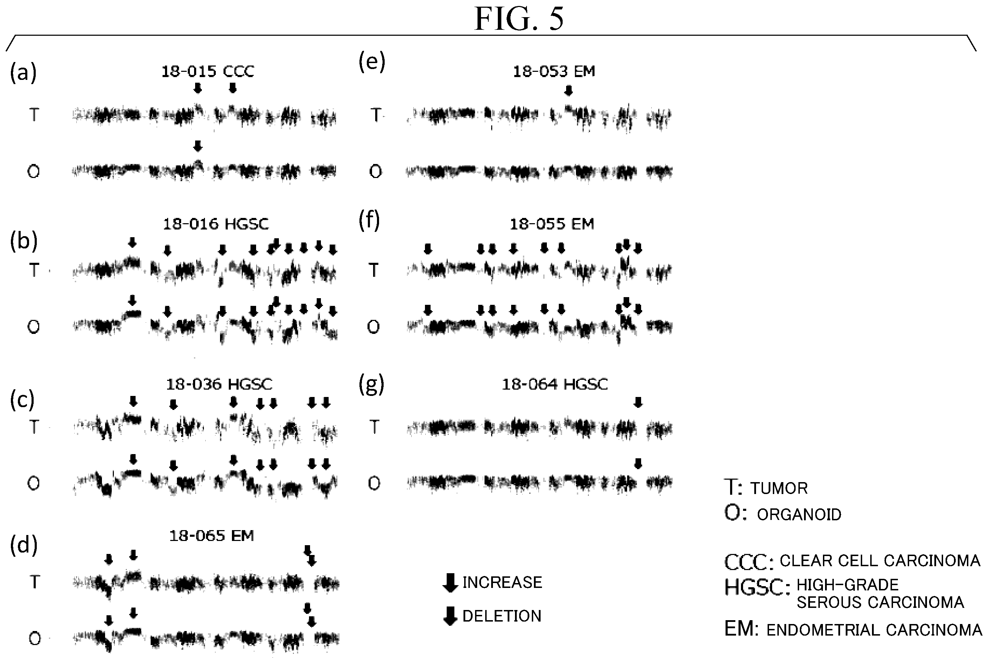

FIG. 5 is a graphic view showing the results of the change in number of copies of chromosomes. The results of CCC18-015 in FIG. 5(a), HGSC18-016 in FIG. 5(b), HGSC18-036 in FIG. 5(c), EM18-065 in FIG. 5(d), EM18-053 in FIG. 5(e), EM18-055 in FIG. 5(f), and EM18-064 in FIG. 5(g) are shown.

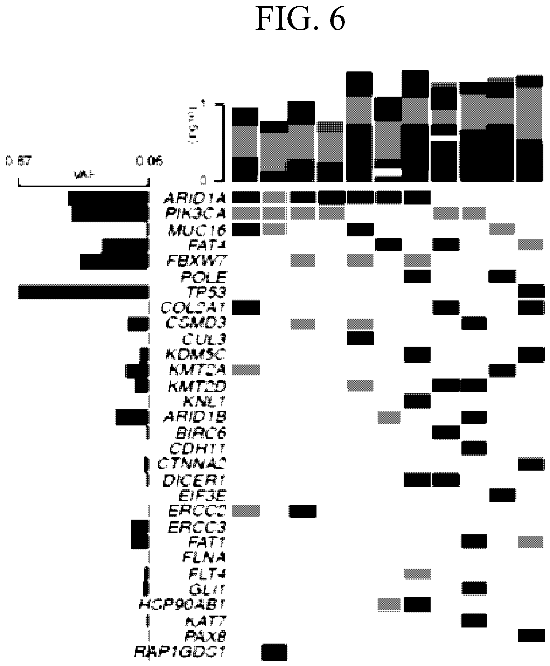

FIG. 6 shows the results of exosome sequencing of ovarian clear cell carcinoma organoids in 11 cases. In the ovarian clear cell carcinoma organoids in 11 cases, the ARID1A mutation was found in 7 cases (63.6%), the PIK3CA mutation was found in 6 cases (54.5%), and the ARID1B mutation was found in 2 cases (18.2%). As in the gene mutation analysis of ovarian clear cell carcinoma in a previous report (Itamochi et al. Br J Cancer, 2017), it has been shown that the results comprehensively cover clear cell carcinoma with both the ARID1A and PIK3CA mutations, clear cell carcinoma with either one of both mutations, and clear cell carcinoma with neither of the mutations, and the ovarian clear cell carcinoma organoids in the 11 cases comprehensively cover the gene mutations of ovarian clear cell carcinoma.

[Experimental Example 4: Sensitivity Test of Ovarian Cancer Organoid to PARP Inhibitor]

A sensitivity test of the ovarian cancer organoid to olaparib was performed. Olaparib which is a kind of PARP inhibitor was prepared at a final concentration of each of 0.0001 $\mu$M, 0.001 $\mu$M, 0.01 $\mu$M, 0.1 $\mu$M, 1 $\mu$M, and 10 $\mu$M, and the ovarian cancer organoid was exposed to olaparib for 3 days at each concentration, and the cell viability was measured by measuring the ATP activity.

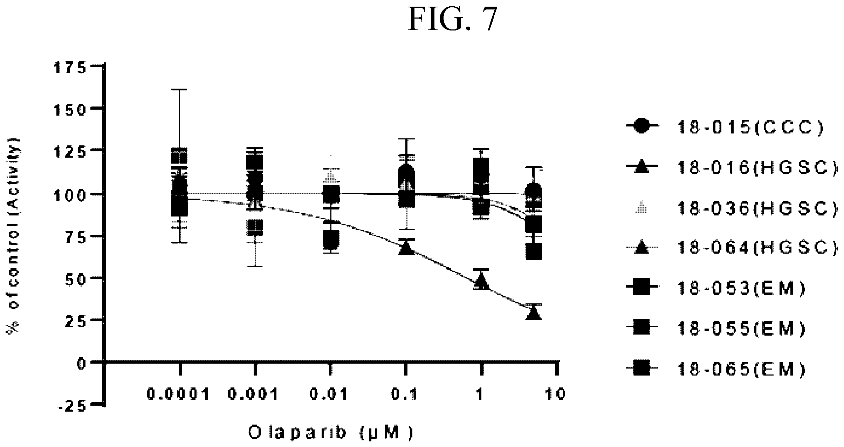

FIG. 7 shows a graphic view showing the cell viability. The vertical axis indicates the cell viability as a ratio to the control, and the horizontal axis indicates the final concentration ($\mu$M) of olaparib.

As shown in FIG. 7, the results showing an overall tendency that the cell viability is reduced depending on the concentration of olaparib for each cell line were obtained. In addition, the results that the cell viability of HGSC18-016 significantly changes were obtained.

CA18-016 is a high-grade serous ovarian cancer having a pathogenic variant (p.L63X) in BRCA1, and HGSC18-016 is also a high-grade serous ovarian cancer organoid having a pathogenic variant in BRCA1. In addition, the other pathological specimens do not have a pathogenic variant in BRCA1. FIG. 7 reflects a clinical result that olaparib is effective against a pathogen having a pathogenic variant in BRCA1, and shows that the established ovarian cancer organoid reflects the characteristics of the pathologenic specimen.

[Experimental Example 5: Transplantation of Ovarian Cancer Organoid into Immunodeficient Mouse]

The ovarian cancer organoid was transplanted into an immunodeficient mouse and the tumor forming ability was evaluated.

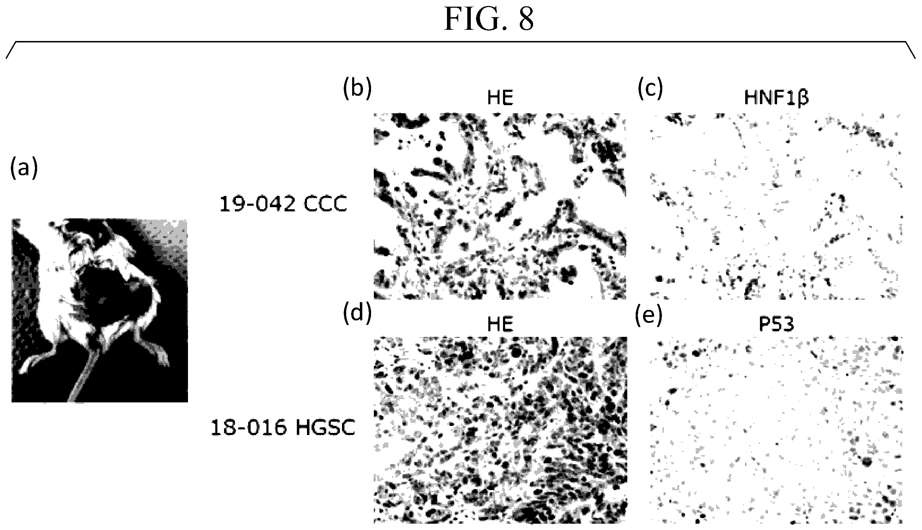

FIG. 8 shows tissue images after transplantation of ovarian cancer organoids into immunodeficient mice, and images of thinly sliced slides that were created from FFPE sections and subjected to HE staining, p53 immunostaining, and HNF1β staining. FIG. 8(a) shows a photographic view of a tissue of an immunodeficient mouse into which an ovarian cancer organoid has been transplanted. For the CCC19-042 of the clear cell carcinoma organoid, FIG. 8(b) shows the HE staining view and FIG. 8(c) shows the HNF1β staining view. For the HGSC18-016 of the serous carcinoma organoid, FIG. 8(d) shows the HE staining view and FIG. 8(e) shows the p53 staining view.

According to FIG. 8, tumor formation was observed in both the clear cell carcinoma organoid and the serous carcinoma organoid. From the results of the HE staining and the immunohistochemical staining, it was also shown that the pathological and immunohistochemical characteristics of the clear cell carcinoma and the serous carcinoma are retained.

(Drug Screening Using Ovarian Cancer Organoid)

[Experimental Example 6: Construction of Assay System]

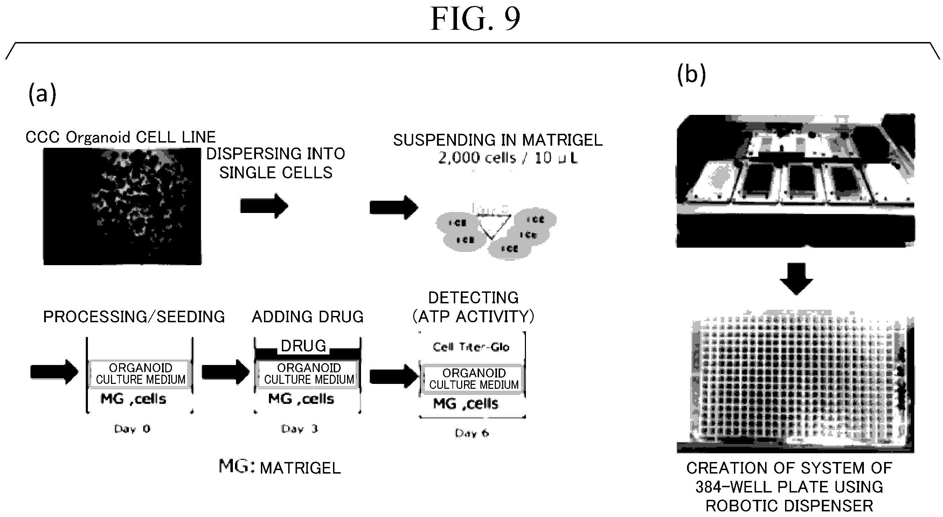

FIG. 9 shows a method for constructing an assay system for performing high-throughput drug screening (HTDS). FIG. 9(a) shows a schematic view of the construction method, and FIG. 9(b) shows a photographic view of the seeding of the cell suspension.

After dispersing the ovarian cancer organoids into single cells, the cells were suspended in Matrigel (manufactured by Thermo Fisher Scientific, Inc.) at a density of 2,000 cells/10 $\mu$l while being ice-cooled. The suspension was seeded in a 384-well plate (product name: Ultra-Low Attachment Surface, 384-Well Low Flange Clear Bottom Black Flat Bottom Plate with Lid, by Corning Inc.) at 10 $\mu$l/well, and then the culture medium described in Experimental Example 2 was laminated at 40 $\mu$l/well to prepare an assay system.

[Experimental Example 7: HTDS Using Compound Library]

After the construction of the assay system, the drug (1 $\mu$M) of the library (Selleck Bioactive compound library (4,650 cpds), Ministry of Education, Culture, Sports, Science and Technology Molecular Profile Support Standard Inhibitor Kit (384 cpds)) was added to the screening system on the 3rd day, and the ATP activity was detected on the 5th day.

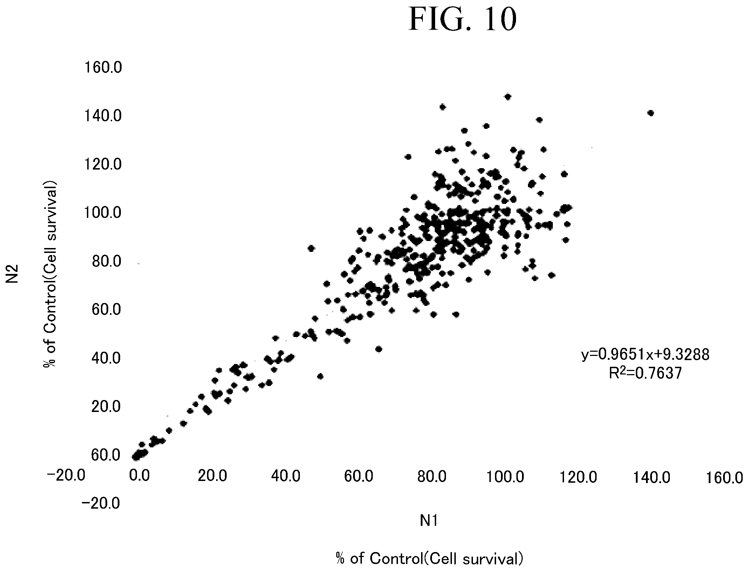

FIG. 10 shows a graphic view of the results of HTDS. The coefficient of determination $R^2$ in the two runs (N = 2) of HTDS was 0.7673, indicating that the screening system was reasonable.

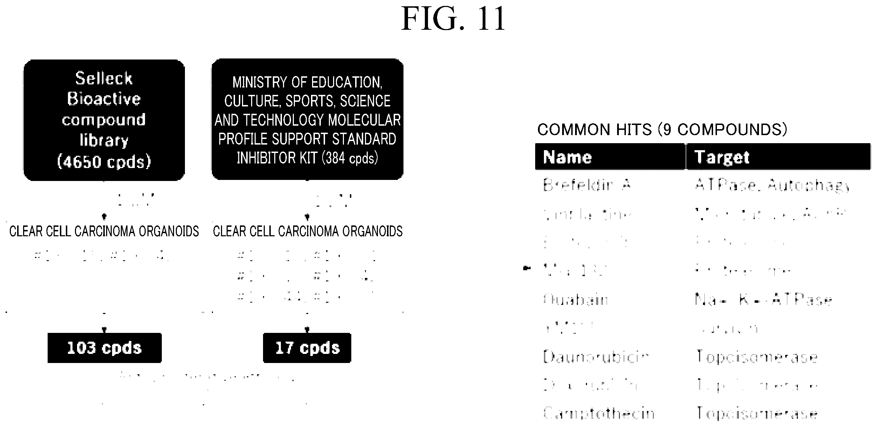

FIG. 11 is a schematic view showing a protocol of HTDS using ovarian clear cell carcinoma organoids. HTDS was performed using a library of 384 standard inhibitors for clear cell carcinoma organoids in 6 cases. In addition, similarly, HTDS was performed on clear cell carcinoma organoids in 2 cases using a library of 4,650 compounds. As a result, 9 compounds were identified as common hits.

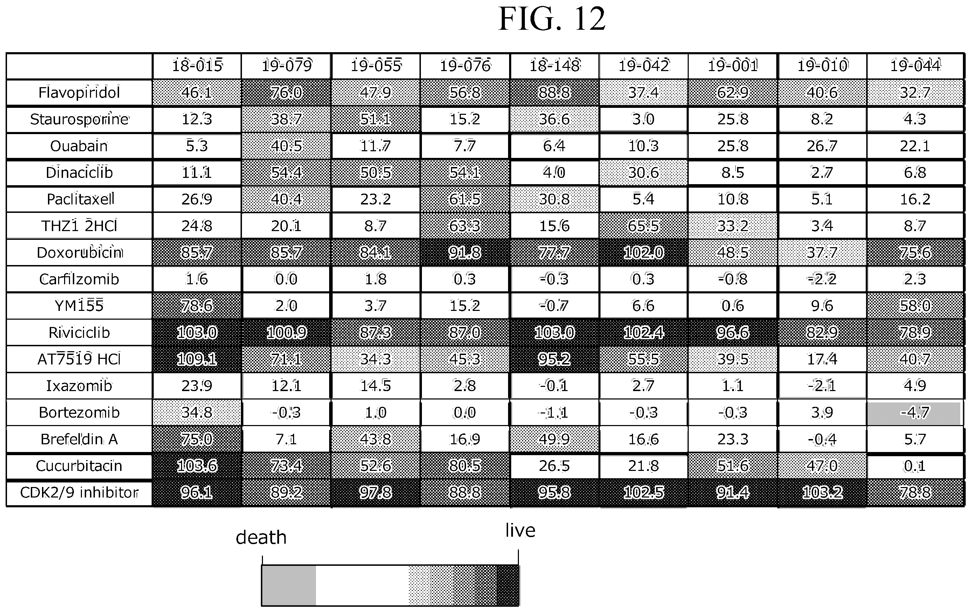

FIG. 12 shows a heat map based on the results of HTDS (0.1 $\mu$M).

(Drug Sensitivity Test of HTDS Hit Compound to Ovarian Clear Cell Carcinoma Organoid)

[Experimental Example 8: Sensitivity Test of Ovarian Clear Cell Carcinoma Organoid to Proteasome Inhibitor]

Based on the above-described results of HTDS, the test focused on the proteasome inhibitors that were found with multiple hits among the 9 hit compounds. A proteasome inhibitor (bortezomib, carfilzomib, and ixazomib), a microtubule inhibitor (paclitaxel), and a platinum formulation (cisplatin and carboplatin) were prepared at each of final concentrations of 0.0001 $\mu$M, 0.001 $\mu$M, 0.01 $\mu$M, 0.1 $\mu$M, 1 $\mu$M, and 10 $\mu$M, the ovarian clear cell carcinoma organoid was exposed to each of the concentrations for 3 days, and the cell viability was measured by measuring the ATP activity.

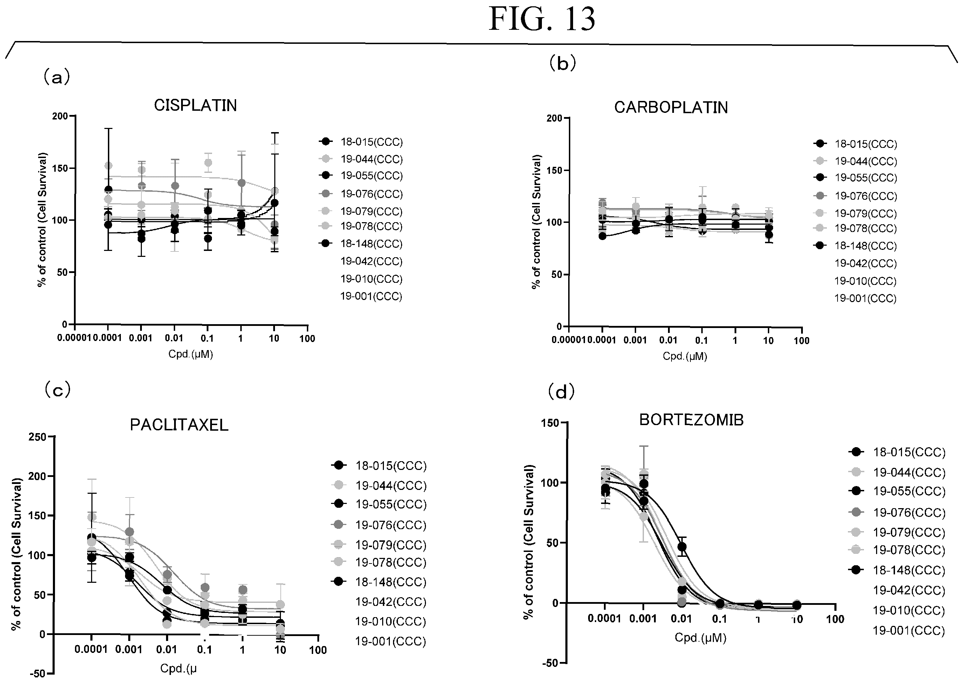

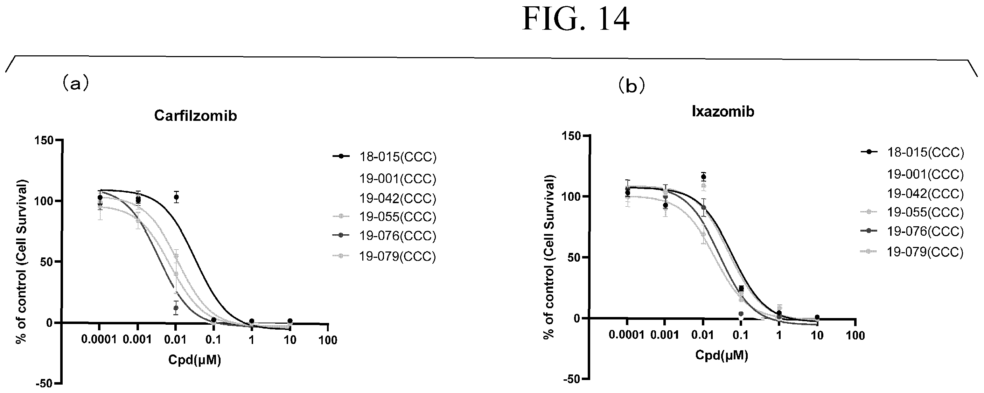

FIGS. 13 and 14 show graphic views of the results of the measured cell viabilities. FIG. 13(a) shows the cell viability of cisplatin, FIG. 13(b) shows the cell viability of carboplatin, FIG. 13(c) shows the cell viability of paclitaxel, FIG. 13(d) shows the cell viability of bortezomib, FIG. 14(a) shows the cell viability of carfilzomib, and FIG. 14(b) shows the cell viability of ixazomib.

According to FIGS. 13 and 14, it was shown that the administration of paclitaxel, which is a microtubule inhibitor, and bortezomib, carfilzomib, and ixazomib, which are proteasome inhibitors, resulted in a significant decrease in cell viability depending on the concentration in all ovarian clear cell carcinoma organoids.

[Experimental Example 9: Transplantation of Ovarian Clear Cell Carcinoma Organoid into Immunodeficient Mouse and Evaluation of Tumor Forming Ability]

The ovarian clear cell carcinoma organoid CCC19-042 was cultured until it reached a diameter of approximately 50 to 100 $\mu$m. The organoid was recovered in a 50 mL tube after removing the culture medium from the plate and then suspending the organoid in a Cell Recovery Solution (Corning, 354235). The solution including the recovered organoid was placed on ice and the Matrigel was depolymerized. After the completion of the reaction, the organoid was washed with a culture medium and resuspended in the Matrigel so that $1 \times 10^6$ cells were present in 100 $\mu$L.

The organoid corresponding to $1 \times 10^6$ cells was transplanted subcutaneously (left shoulder) in an immunodeficient mouse, and the tumor forming ability was evaluated on the 7th day after the transplantation. In addition, a thinly sliced slide was created from an FFPE section of the tumor and subjected to HE staining and HNF1$\beta$ immunostaining.

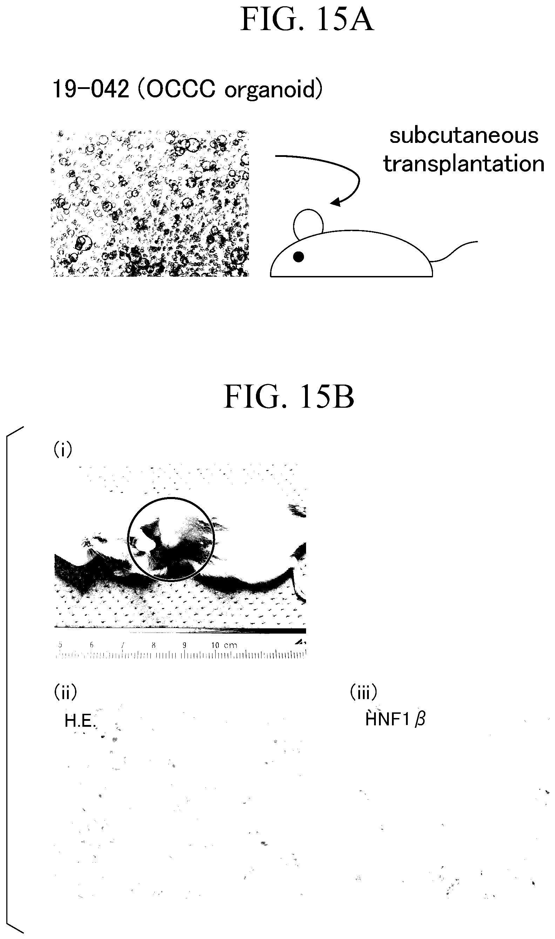

FIG. 15A shows a microscopic observation image of an ovarian clear cell carcinoma organoid CCC19-042. The observation was performed in the same manner as in Experimental Example 2 and the scale in the drawing is 100 $\mu$m.

FIG. 15B(i) shows a visual view of a tissue image of the immunodeficient mouse 1 week after the transplantation of the ovarian clear cell carcinoma organoid CCC19-042. A portion circled by a line in the photographic view is the tissue of the transplanted portion.

FIG. 15B(ii) shows the HE staining view and FIG. 15B(iii) shows the HNF1$\beta$ immunostaining view for the tumor. The HE staining and the HNF1$\beta$ staining were performed by the same operation as in Experimental Example 2.

According to FIGS. 15A and 15B, tumor formation of the ovarian clear cell carcinoma organoid was observed. From the results of the HE staining and the immunohistochemical staining, it was also shown that the pathological and immunohistochemical characteristics of the clear cell carcinoma are retained.

[Experimental Example 10: Drug Sensitivity Test for Immunodeficient Mouse Transplanted with Ovarian Clear Cell Carcinoma Organoid]

With regard to the immunodeficient mouse transplanted with the ovarian clear cell carcinoma organoid shown in Experimental Example 9, administration, measurement of a weekly body weight, and calculation of a tumor volume were performed from the 7th day after transplantation. The administration of the drug to the mouse was carried out by intraperitoneally administering DMSO (vehicle) or bortezomib at 1 mg/kg twice a week with an interval of 3 days.

The tumor volume was calculated by the following expression.

$$\text{Tumor volume (mm}^3) = \text{major axis (mm)} \times \{\text{minor axis (mm)}\}^2 \times 1/2.$$

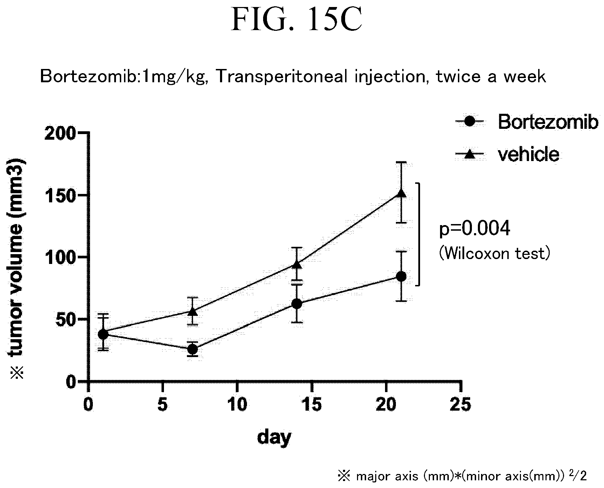

FIG. 15C shows each tumor volume of a mouse per week in a case where a vehicle and bortezomib were administered to the mouse.

According to FIG. 15C, the administration of bortezomib, which is a proteasome inhibitor, also resulted in a decrease in tumor volume in the ovarian clear cell carcinoma organoid that had been transplanted into the immunodeficient mouse.

A proteasome inhibitor such as bortezomib is a drug used for the treatment of multiple myeloma. In general, tumor cells actively perform protein synthesis to maintain the biological functions, and a proteasome-ubiquitin pathway for processing proteins that is no longer necessary within the cells develops. The mechanism of action of the proteasome inhibitor is diverse, but it is considered that the proteasome inhibition causes a disruption in the balance of endoplasmic reticulum associated protein degradation (ERAD), leading to the accumulation of misfolded proteins, ultimately to apoptosis. Multiple myeloma is a tumor of plasma cells that secrete a large amount of abnormal immunoglobulins, and the cytoplasm is rich in endoplasmic reticulum. Clear cell carcinoma is also characterized by a rich amount of endoplasmic reticulum in the cell as well as a rich amount of glycogen in the cell (Silverberg SG. Ultrastructure and histogenesis of clear cell carcinoma of the ovary. Am J Obstet Gynecol 1973; 115: 394-400.), and both the multiple myeloma and the clear cell carcinoma are often complicated by thrombosis. Based on this, the clear cell carcinoma and the multiple myeloma have common characteristics.

In the endoplasmic reticulum, the endoplasmic reticulum stress response (unfolded protein response: UPR) includes an IRE1$\alpha$ pathway, a PERK pathway, and an ATF6 pathway, which are involved in the processing of proteins under normal conditions but induce cell death in a case where the endoplasmic reticulum stress continues.

The clear cell carcinoma organoid, the multiple myeloma (MMRF CoMMpass Study (TCGA)), and the normal ovary (GTEx Analysis V8) were compared by single-sample Gene Set Enrichment Analysis (ssGSEA).

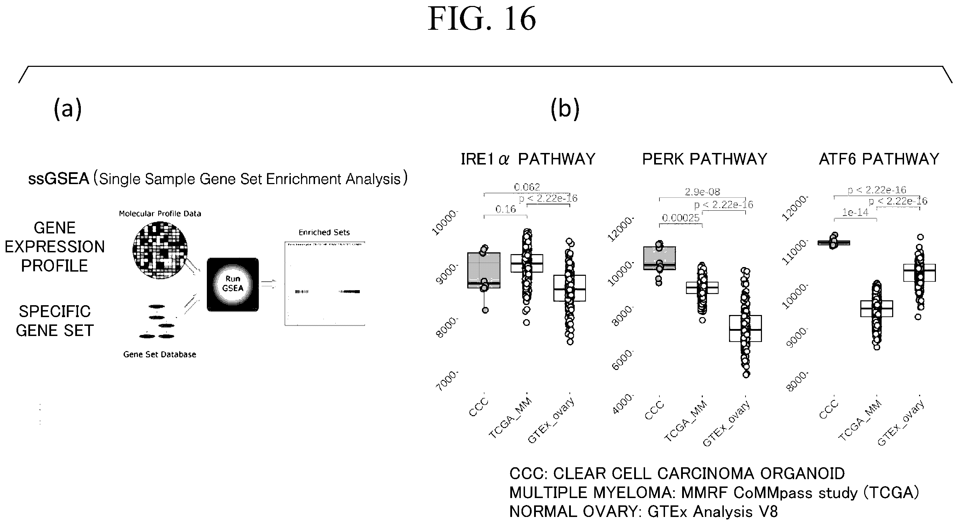

FIG. 16(a) shows the outline of ssGSEA. FIG. 16(b) shows a graphic view of gene expression on the IRE1$\alpha$ pathway, the PERK pathway, and the ATF pathway.

According to the views, the PERK pathway-related gene expression was significantly higher in the clear cell organoid, followed by the multiple myeloma and the normal ovary. In the ATF6 pathway, the clear cell carcinoma organoid was also more upregulated than the multiple myeloma and the normal ovary. Based on this, it is presumed that the UPR is upregulated in the clear cell carcinoma, and the proteasome inhibitor has exerted an effective response as in the multiple myeloma.

[Experimental Example 11: Sensitivity Test of Ovarian Clear Cell Carcinoma Organoid to CDK 1, 2, 5, and 9 Inhibitor]

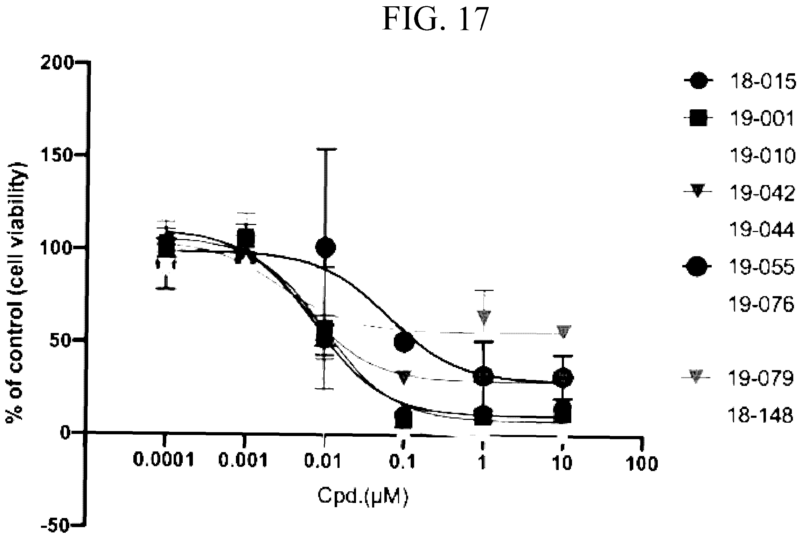

For the CDK 1, 2, 5, and 9 inhibitor (dinaciclib) that was hit in the 9 compounds that were hit more in HTDS, the cell viability was measured by measuring an ATP activity in the same manner as in Experimental Example 8. The results are shown in FIG. 17.

As shown in FIG. 17, the results showing an overall tendency that the cell viability decreased depending on the concentration of dinaciclib for each cell line could be obtained.

Dinaciclib is also known to inhibit UPR, which is presumably one of the reasons why dinaciclib is effective against clear cell carcinoma.

INDUSTRIAL APPLICABILITY

According to the present invention, a pharmaceutical composition that is effective for the treatment of ovarian clear cell carcinoma can be obtained.

**Claims**

1. A therapeutic agent for ovarian clear cell carcinoma, comprising, as an active ingredient:
   a proteasome inhibitor.

2. The therapeutic agent for ovarian clear cell carcinoma according to Claim 1,
   wherein the proteasome inhibitor is a substance that reversibly or irreversibly binds to a 20s $\beta$5 unit of a proteasome and inhibits a chymotrypsin-like activity.

3. The therapeutic agent for ovarian clear cell carcinoma according to Claim 1,
   wherein the proteasome inhibitor is a substance that irreversibly binds to a 20s $\beta$5 unit of a proteasome and inhibits a chymotrypsin-like activity.

4. The therapeutic agent for ovarian clear cell carcinoma according to Claim 2,
   wherein the proteasome is a 26s proteasome.

5. The therapeutic agent for ovarian clear cell carcinoma according to Claim 1,
   wherein a content proportion of the proteasome inhibitor included in the active ingredient is 80% by mass or more, 90% by mass or more, or 100% by mass.

6. The therapeutic agent for ovarian clear cell carcinoma according to Claim 1, wherein the ovarian clear cell carcinoma has cisplatin resistance, carboplatin resistance, or both the cisplatin resistance and the carboplatin resistance.

7. The therapeutic agent for ovarian clear cell carcinoma according to Claim 1,
   wherein the ovarian clear cell carcinoma expresses calnexin, ero1La, CHOP, PDI, PERK, BiP, or IRE1$\alpha$.

8. The therapeutic agent for ovarian clear cell carcinoma according to Claim 7,
   wherein the ovarian clear cell carcinoma expresses calnexin and PDI.

FIG. 1

4TH DAY  7TH DAY  14TH DAY

18-015
CLEAR CELL
CARCINOMA

18-036
HIGH-GRADE SEROUS
CARCINOMA

18-053
ENDOMETRIAL
CARCINOMA

# FIG. 2

EP 4 566 628 A1

FIG. 3

FIG. 4

# FIG. 5

(a) 18-015 CCC
T
O

(b) 18-016 HGSC
T
O

(c) 18-036 HGSC
T
O

(d) 18-065 EM
T
O

(e) 18-053 EM
T
O

(f) 18-055 EM
T
O

(g) 18-064 HGSC
T
O

T: TUMOR
O: ORGANOID

↓ INCREASE
↓ DELETION

CCC: CLEAR CELL CARCINOMA
HGSC: HIGH-GRADE
       SEROUS CARCINOMA
EM: ENDOMETRIAL CARCINOMA

EP 4 566 628 A1

# FIG. 6

# FIG. 7

# FIG. 8

(a)

(b) HE
19-042 CCC

(c) HNF1β

(d) HE
18-016 HGSC

(e) P53

EP 4 566 628 A1

# FIG. 9

(a)

CCC Organoid CELL LINE → DISPERSING INTO SINGLE CELLS → SUSPENDING IN MATRIGEL 2,000 cells / 10 µL

PROCESSING/SEEDING → ADDING DRUG → DETECTING (ATP ACTIVITY)

DRUG

ORGANOID CULTURE MEDIUM
MG ,cells
Day 0

ORGANOID CULTURE MEDIUM
MG ,cells
Day 3

Cell Titer-Glo
ORGANOID CULTURE MEDIUM
MG ,cells
Day 6

MG: MATRIGEL

(b)

CREATION OF SYSTEM OF 384-WELL PLATE USING ROBOTIC DISPENSER

EP 4 566 628 A1

FIG. 10

# FIG. 11

Selleck Bioactive compound library (4650 cpds)

MINISTRY OF EDUCATION, CULTURE, SPORTS, SCIENCE AND TECHNOLOGY MOLECULAR PROFILE SUPPORT STANDARD INHIBITOR KIT (384 cpds)

CLEAR CELL CARCINOMA ORGANOIDS   CLEAR CELL CARCINOMA ORGANOIDS

103 cpds     17 cpds

COMMON HITS (9 COMPOUNDS)

| Name | Target |
| --- | --- |
| Brefeldin A | ATPase, Autophagy |
|  |  |
|  |  |
|  |  |
| Ouabain | Na+ K+ ATPase |
|  |  |
| Daunorubicin | Topoisomerase |
|  |  |
| Camptothecin | Topoisomerase |

# FIG. 12

| | 18-015 | 19-079 | 19-055 | 19-076 | 18-148 | 19-042 | 19-001 | 19-010 | 19-044 |
|---|---|---|---|---|---|---|---|---|---|
| Flavopiridol | 46.1 | 76.0 | 47.9 | 56.8 | 88.8 | 37.4 | 62.9 | 40.6 | 32.7 |
| Staurosporine | 12.3 | 38.7 | 51.1 | 15.2 | 36.6 | 3.0 | 25.8 | 8.2 | 4.3 |
| Ouabain | 5.3 | 40.5 | 11.7 | 7.7 | 6.4 | 10.3 | 25.8 | 26.7 | 22.1 |
| Dinaciclib | 11.1 | 54.4 | 50.5 | 54.1 | 4.0 | 30.6 | 8.5 | 2.7 | 6.8 |
| Paclitaxell | 26.9 | 40.4 | 23.2 | 61.5 | 30.8 | 5.4 | 10.8 | 5.1 | 16.2 |
| THZ1 2HCl | 24.8 | 20.1 | 8.7 | 63.3 | 15.6 | 65.5 | 33.2 | 3.4 | 8.7 |
| Doxorubicin | 85.7 | 85.7 | 84.1 | 91.8 | 77.7 | 102.0 | 48.5 | 37.7 | 75.6 |
| Carfilzomib | 1.6 | 0.0 | 1.8 | 0.3 | -0.3 | 0.3 | -0.8 | -2.2 | 2.3 |
| YM155 | 78.6 | 2.0 | 3.7 | 15.2 | -0.7 | 6.6 | 0.6 | 9.6 | 58.0 |
| Riviciclib | 103.0 | 100.9 | 87.3 | 87.0 | 103.0 | 102.4 | 96.6 | 82.9 | 78.9 |
| AT7519 HCl | 109.1 | 71.1 | 34.3 | 45.3 | 95.2 | 55.5 | 39.5 | 17.4 | 40.7 |
| Ixazomib | 23.9 | 12.1 | 14.5 | 2.8 | -0.1 | 2.7 | 1.1 | -2.1 | 4.9 |
| Bortezomib | 34.8 | -0.3 | 1.0 | 0.0 | -1.1 | -0.3 | -0.3 | 3.9 | -4.7 |
| Brefeldin A | 75.0 | 7.1 | 43.8 | 16.9 | 49.9 | 16.6 | 23.3 | -0.4 | 5.7 |
| Cucurbitacin | 103.6 | 73.4 | 52.6 | 80.5 | 26.5 | 21.8 | 51.6 | 47.0 | 0.1 |
| CDK2/9 inhibitor | 96.1 | 89.2 | 97.8 | 88.8 | 95.8 | 102.5 | 91.4 | 103.2 | 78.8 |

death      live

EP 4 566 628 A1

FIG. 13

# FIG. 14

(a)

**Carfilzomib**

(b)

**Ixazomib**

# FIG. 15A

19-042 (OCCC organoid)

subcutaneous
transplantation

# FIG. 15B

(i)

(ii)

H.E.

(iii)

HNF1 $\beta$

# FIG. 15C

Bortezomib:1mg/kg, Transperitoneal injection, twice a week

— Bortezomib
— vehicle

p=0.004
(Wilcoxon test)

※ major axis (mm)*(minor axis(mm)) $^{2}/2$

# FIG. 16

**(a)**

**(b)**

CCC: CLEAR CELL CARCINOMA ORGANOID
MULTIPLE MYELOMA: MMRF CoMMpass study (TCGA)
NORMAL OVARY: GTEx Analysis V8

# FIG. 17

EP 4 566 628 A1

## INTERNATIONAL SEARCH REPORT

International application No.

**PCT/JP2023/028482**

| A. | CLASSIFICATION OF SUBJECT MATTER |
| --- | --- |

*A61K 45/00*(2006.01)i; *A61P 15/00*(2006.01)i; *A61P 35/00*(2006.01)i; *A61K 31/69*(2006.01)i
FI:   A61K45/00; A61P15/00; A61P35/00; A61K31/69

According to International Patent Classification (IPC) or to both national classification and IPC

| B. | FIELDS SEARCHED |
| --- | --- |

Minimum documentation searched (classification system followed by classification symbols)

A61K45/00; A61P15/00; A61P35/00; A61K31/69

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | WO 2021/113743 A1 (THE JOHNS HOPKINS UNIVERSITY) 10 June 2021 (2021-06-10) pp. 35, 36 | 1, 5-8 |
| Y | pp. 35, 36 | 2-4 |
| Y | ROETEN, Margot S. F. et al. Positioning of proteasome inhibitors in therapy of solid malignancies. Cancer Chemotherapy and Pharmacology. 2018, vol. 81, pp. 227-243, https://doi.org/10.1007/s00280-017-3489-0 table 1 | 2-4 |
| Y | AGHAJANIAN, C. et al. Phase I Trial of Bortezomib and Carboplatin in Recurrent Ovarian or Primary Peritoneal Cancer. Journal of Clinical Oncology. 2005, vol. 23, no. 25, pp. 5943-5949, DOI: 10.1200/JCO.2005.16.006 abstract | 2-4 |

☐ Further documents are listed in the continuation of Box C.       ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **12 October 2023** | **24 October 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2023/028482**

| Patent document cited in search report | Publication date (day/month/year) | Patent family member(s) | Publication date (day/month/year) |
|---|---|---|---|
| WO 2021/113743 A1 | 10 June 2021 | US 2023/0045142 A1 paragraphs [0167]-[0169] | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2022125006 A

- US 20140364375 A

**Non-patent literature cited in the description**

- Structure-function analyses of candidate small molecule RPN13 inhibitors with antitumor properties. *PLoS One.*, 15 January 2020, vol. 15 (1), e0227727
- Caution over use of ES2 as a model of ovarian clear cell carcinoma. *J Clin Pathol.*, October 2014, vol. 67 (10), 921-2
- Evaluating cell lines as tumour models by comparison of genomic profiles. *Nat Commun.*, 2013, vol. 4, 2126
- *CHEMICAL ABSTRACTS*, 179324-69-7
- *CHEMICAL ABSTRACTS*, 133407-82-6
- *CHEMICAL ABSTRACTS*, 868540-17-4
- *CHEMICAL ABSTRACTS*, 1239908-20-3
- *CHEMICAL ABSTRACTS*, 1072833-77-2
- *CHEMICAL ABSTRACTS*, 960374-59-8

- *CHEMICAL ABSTRACTS*, 935888-69-0
- *CHEMICAL ABSTRACTS*, 847499-27-8
- *CHEMICAL ABSTRACTS*, 34157-83-0
- *CHEMICAL ABSTRACTS*, 134381-21-8
- *CHEMICAL ABSTRACTS*, 517-89-5
- *CHEMICAL ABSTRACTS*, 1624602-30-7
- *CHEMICAL ABSTRACTS*, 548-19-6
- *CHEMICAL ABSTRACTS*, 437742-34-2
- *CHEMICAL ABSTRACTS*, 1617495-03-0
- *CHEMICAL ABSTRACTS*, 1401223-22-0
- *CHEMICAL ABSTRACTS*, 18797-80-3
- **MITRA AK et al.** *Gynecol Oncol.*, 2015
- **ITAMOCHI et al.** *Br J Cancer*, 2017
- **SILVERBERG SG**. Ultrastructure and histogenesis of clear cell carcinoma of the ovary. *Am J Obstet Gynecol*, 1973, vol. 115, 394-400